(19) Europäisches Patentamt / European Patent Office / Office européen des brevets

(11) **EP 2 782 500 B1**

(12) **FASCICULE DE BREVET EUROPEEN**

(45) Date de publication et mention de la délivrance du brevet:
**12.08.2015 Bulletin 2015/33**

(21) Numéro de dépôt: **12786976.6**

(22) Date de dépôt: **12.11.2012**

(51) Int Cl.:
**A61B 5/053** (2006.01)

(86) Numéro de dépôt international:
**PCT/EP2012/072388**

(87) Numéro de publication internationale:
**WO 2013/075963 (30.05.2013 Gazette 2013/22)**

(54) **PROCEDE D'ANALYSE ÉLECTROPHYSIOLOGIQUE DE FIABILITE ACCRUE**

VERFAHREN ZUR DURCHFÜHRUNG EINER ELEKTROPHYSIOLOGISCHEN ANALYSE MIT ERHÖHTER ZUVERLÄSSIGKEIT

METHOD FOR PERFORMING AN ELECTROPHYSIOLOGICAL ANALYSIS WITH INCREASED RELIABILIT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **21.11.2011 FR 1160601**

(43) Date de publication de la demande:
**01.10.2014 Bulletin 2014/40**

(73) Titulaire: **Impeto Medical**
**75014 Paris (FR)**

(72) Inventeur: **BRUNSWICK, Philippe**
**F-75014 Paris (FR)**

(74) Mandataire: **Regimbeau**
**20, rue de Chazelles**
**75847 Paris Cedex 17 (FR)**

(56) Documents cités:
**FR-A1- 2 912 893**

- **BRUNSWICK P ET AL: "Use of Ni electrodes chronoamperometry for improved diagnostics of diabetes and cardiac diseases", 2007 ANNUAL INTERNATIONAL CONFERENCE OF THE IEEE ENGINEERING IN MEDICINE AND BIOLOGY SOCIETY : [EMBC '07] ; LYON, FRANCE, 22 - 26 AUGUST 2007 ; [IN CONJUNCTION WITH THE BIENNIAL CONFERENCE OF THE SOCIÉTÉ FRANÇAISE DE GÉNIE BIOLOGIQUE ET MÉDICAL (SFGB, 22 août 2007 (2007-08-22), pages 4544-4547, XP031337229, ISBN: 978-1-4244-0787-3**
- **KAMEL KHALFALLAH ET AL: "Noninvasive Galvanic Skin Sensor for Early Diagnosis of Sudomotor Dysfunction: Application to Diabetes", IEEE SENSORS JOURNAL, IEEE SERVICE CENTER, NEW YORK, NY, US, vol. 12, no. 3, 30 décembre 2010 (2010-12-30), pages 456-463, XP011408126, ISSN: 1530-437X, DOI: 10.1109/JSEN.2010.2103308**

EP 2 782 500 B1

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

**Description**

DOMAINE DE L'INVENTION

L'invention concerne de manière générale le domaine de l'électrochimie, et plus particulièrement le domaine de l'analyse électrophysiologique du corps humain, en vue par exemple de détecter des pathologies.
L'invention est notamment applicable à l'évaluation de la fonction sudorale du corps humain.

ART ANTERIEUR

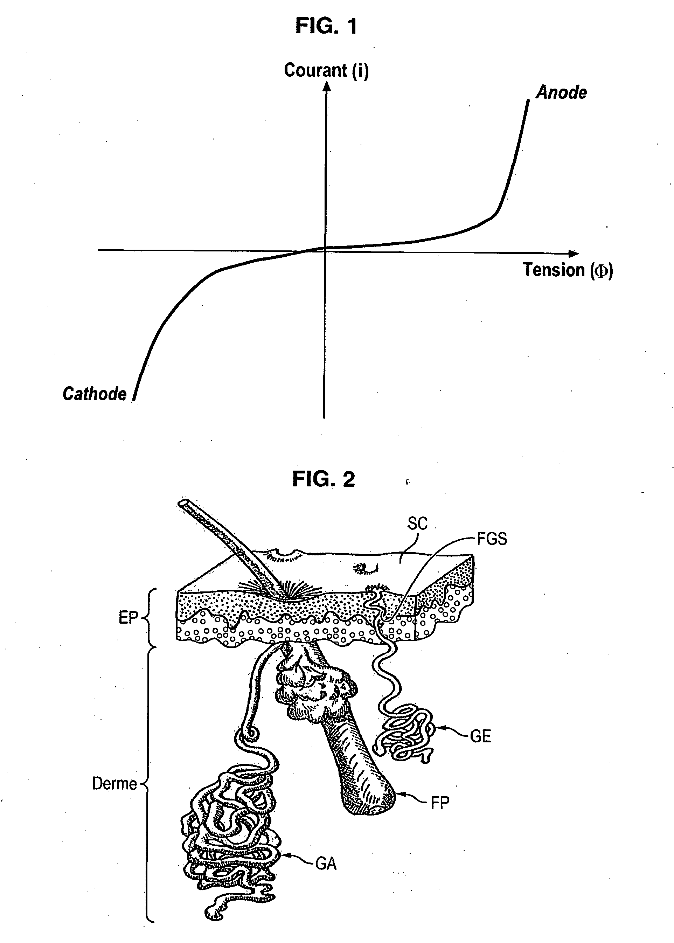

La Demanderesse a déjà proposé dans le brevet FR 2 912 893 un système d'analyse électrophysiologique comprenant une série d'électrodes destinées à être positionnées en différentes régions du corps d'un patient, une source de tension continue, adaptée pour générer des créneaux de tension continue ajustable, et un circuit de commutation, agencé pour sélectivement relier une paire d'électrodes dites actives à la source de tension, lesdites électrodes actives constituant une anode et une cathode, et pour connecter au moins une autre électrode en haute impédance.
La tension appliquée par la source de tension sur les électrodes permet de générer dans la couche externe de la peau un courant électrophysiologique dont l'étude de certaines caractéristiques peut indiquer certaines pathologies.
Ainsi par exemple, une faible pente de la courbe tension-courant peut être un indice, chez un patient diabétique, d'une neuropathie diabétique, comme décrit dans le document « Gin H, et al. Non-invasive and quantitative assessment of sudomotor function for peripheral diabetic neuropathy evaluation. Diabetes Metab (2011), doi: 10.1016/j.diabet.2011.05.003 ».
Pour permettre une bonne interprétation des mesures acquises grâce à ce système, il faut s'assurer de la qualité et de la pertinence de cette mesure. Il faut donc, d'une part s'assurer que le modèle utilisé pour lier les mesures au comportement effectif du corps est fiable et pertinent. D'autre part, il faut réaliser les mesures les plus précises possibles, en limitant au maximum les incertitudes et les biais liés au système de mesure.
inoxidable, utilisée comme anode ou cathode, dans des solutions mimant la sueur, qui est représenté en figure 1.
De façon connue en soi, on assiste, respectivement à l'anode et à la cathode, à des réactions d'oxydation et de réduction liées à l'application d'un potentiel sur ces électrodes.
Coté anode, les chlorures participent à l'oxydation de l'anode, et un mur se forme au-delà d'un certain seuil en tension.
Coté cathode, on assiste à une réduction d'éventuels oxydes et à la réduction de l'eau présente dans la sueur, occasionnant un dégagement de hydrogène. La cathode est alors réduite, ce qui conduit également à la formation d'un mur en deçà d'un seuil en tension.
La position de ces murs d'oxydation et de réduction sur le voltammogramme dépend de l'électrode (composition, taux d'utilisation) ainsi que des concentrations en électrolytes occasionnant les réactions d'oxydo-réduction.
Le voltammogramme représente ainsi le comportement intrinsèque de l'électrode, c'est-à-dire le courant maximum qu'elle peut transmettre pour un potentiel donné, et qui est gouverné par le transfert de charge entre l'électrode et l'électrolyte.
On comprend que l'oxydation de l'anode implique la création progressive d'une surtension qui biaise les mesures.
Il est donc nécessaire d'obtenir des mesures plus précises que celles obtenues jusqu'ici avec le système décrit ci-avant, en particulier en corrigeant les biais de mesure liés aux électrodes.

RESUME DE L'INVENTION

Un des buts de l'invention est de pallier aux problèmes évoqués ci-avant en proposant un procédé d'analyse électrophysiologique au cours duquel les mesures acquises sont plus fiables et pertinentes que dans l'art antérieur. En particulier, l'invention a pour but de corriger les biais pouvant survenir au cours de la mesure.
Un autre but de l'invention est de proposer un modèle précis des phénomènes électrochimiques occasionnés lors de l'application d'un potentiel par des électrodes, afin de rendre l'interprétation des mesures plus fiable.
A cet effet, l'invention propose un procédé d'analyse électrophysiologique mis en oeuvre dans un système comprenant :

une série d'électrodes, destinées à être placées en différentes régions du corps humain,
une source de tension continue, commandée pour engendrer des créneaux de tension continue,
un circuit de commutation, agencé pour sélectivement relier une paire d'électrodes dites actives à la source de tension, lesdites électrodes actives constituant une anode et une cathode, et pour connecter au moins une autre électrode passive en haute impédance servant à mesurer le potentiel atteint par le corps, et

un circuit de mesure agencé pour relever des données représentatives du courant dans les électrodes activés et des potentiels sur au moins certaines électrodes connectées en haute impédance en réponse à l'application des créneaux, lesdites données permettant de déterminer une valeur de la conductance électrochimique de la peau, le procédé comprenant au moins une étape de mesure au cours de laquelle la source de tension continue ajustable applique à l'anode une série desdits créneaux de tension continue, et au cours de laquelle le circuit de mesure relève lesdites données,

le procédé étant caractérisé en ce qu'il comprend en outre une étape préalable à l'étape de mesure, au cours de laquelle une électrode qui est connectée en haute impédance au cours de l'étape de mesure est régénérée en étant connectée à la source de tension en tant que cathode.

Avantageusement, mais facultativement, le procédé selon l'invention comprend en outre au moins l'une des caractéristiques suivantes :

- au cours de l'étape de régénération, la cathode est soumise à un potentiel continu compris entre -1 et -4 V, préférablement entre -3 et -3.5 V, la source de tension délivre un créneau de tension d'une durée comprise entre 5 secondes et une minute, préférablement entre 10 et 30 secondes, ou elle délivre des créneaux de tension identique ou variable d'un créneau à l'autre, la durée cumulée des créneaux étant alors comprise entre 5 secondes et une minute, préférablement entre 10 et 30 secondes.
- au cours d'une étape de régénération, le circuit de mesure relève les données représentatives du courant dans les électrodes actives, de leurs potentiels, et des potentiels sur au moins certaines électrodes connectées en haute impédance.
- Le procédé peut comprendre une étape de régénération supplémentaire, préalable à l'étape de mesure, au cours de laquelle une autre électrode connectée en haute impédance au cours de l'étape de mesure est régénérée en étant connectée à la source de tension en tant que cathode. Le procédé peut alors comprendre une étape de calcul de la moyenne des potentiels des électrodes régénérées connectées en haute impédance.
- les créneaux de tension appliqués lors de l'étape de mesure ont une durée supérieure ou égale à 0,2 seconde.
- la tension continue appliquée à l'anode est inférieure à 10V, et préférablement comprise entre 0V et 4V.
- la source de tension délivre, au cours de l'étape de mesure, des créneaux de tensions variables d'un créneau à l'autre.
- chaque électrode est positionnée sur une zone parmi le groupe suivant : main droite, main gauche, pied droit, pied gauche, côté droit du front, côté gauche du front.
- au cours du procédé, on calcule localement la conductance électrochimique de la peau au niveau de l'anode et de la cathode à partir des données relevées respectivement à l'anode et à la cathode.
- la conductance électrochimique de la peau déterminée à l'anode ou à la cathode est la pente de la courbe mesurée dans un graphe courant-tension pour des tensions appliquées à l'anode inférieure à 2 V, indépendamment de toute surtension.
- le procédé peut en outre comprendre une étape intermédiaire, entre l'étape de régénération et l'étape de mesure, au cours de laquelle on mesure une différence de tension entre une électrode non régénérée connectée en haute impédance, et une électrode régénérée connectée en haute impédance, ladite différence permettant de déterminer une valeur de surtension à l'électrode non régénérée, et, au cours de l'étape de mesure, on connecte en tant qu'anode l'électrode non régénérée dont on a mesuré la surtension. Le procédé peut alors comprendre une étape de détermination, à partir de la surtension de l'électrode haute impédance non régénérée, d'une correction à appliquer aux valeurs mesurées au cours de l'étape de mesure. Dans ce cas, l'électrode régénérée connectée en haute impédance au cours de l'étape intermédiaire a préalablement été connectée en tant que cathode, et le procédé comprend une étape dans laquelle on retranche du potentiel mesuré à l'anode au cours de l'étape de mesure la surtension déterminée au cours de l'étape intermédiaire.
- Le procédé comprend une étape de régénération supplémentaire, entre l'étape de régénération et l'étape de mesure, au cours de laquelle l'anode et la cathode sont respectivement les mêmes que celles de l'étape de mesure. La conductance de la peau au niveau de la cathode est alors obtenue en divisant le courant mesuré à la cathode par la différence de potentiel entre la cathode et une électrode régénérée connectée en haute impédance. Le cas échéant, on estime une surtension à l'anode par la valeur du potentiel à l'anode extrapolé quand le courant s'annule, et on obtient la conductance de la peau au niveau de l'anode en divisant le courant mesuré à l'anode par la différence entre le potentiel de l'anode retranché de ladite surtension et le potentiel d'une électrode régénérée connectée en haute impédance.
- la durée cumulée des créneaux de tension appliqués lors de l'étape de mesure est comprise entre 5 secondes et une minute, préférablement entre 10 et 30 secondes,
- la durée cumulée des créneaux de tension appliqués lors de l'étape de mesure étant supérieure ou égale à la durée du créneau appliqué lors d'une étape de régénération.
- Au cours du procédé, on estime une surtension à l'anode par la valeur du potentiel à l'anode extrapolé quand le courant s'annule, et on obtient la conductance de la peau au niveau de l'anode en divisant le courant mesuré à l'anode par la différence entre le potentiel de l'anode retranché de ladite surtension et le potentiel d'une électrode régénérée connectée en haute impédance.

- Le procédé comprend au moins une étape de régénération supplémentaire, préalable à l'étape de mesure, au cours de laquelle l'électrode utilisée en tant qu'anode au cours de l'étape de mesure est régénérée en étant connectée à la source de tension en tant que cathode, le procédé pouvant alors comprendre un cycle d'étapes de régénération d'électrodes et d'étapes de mesure, dans lequel, pour chaque étape de mesure, l'anode et au moins une électrode connectée en haute impédance ont été préalablement régénérées au cours d'au moins deux étapes de régénération ou de mesure. Dans ce cas, la cathode utilisée au cours d'une étape de mesure est commutée comme anode au cours d'une étape de mesure subséquente. Si le procédé est mis en oeuvre dans un système comprenant quatre électrodes, pour chaque étape de mesure, l'anode et les deux électrodes connectées en haute impédance ont été préalablement régénérées au cours d'étapes de régénération ou de mesure.
- Au cours du procédé, on mesure une conductance de la peau à l'anode en divisant le courant mesuré à l'anode par la différence de potentiel entre l'anode retranché de la surtension et une électrode régénérée connectée en haute impédance, et dans lequel la conductance électrochimique de la peau ainsi obtenue correspond à la conductance électrique des parois des glandes sudoripares eccrines en contact avec les électrodes actives.

L'invention concerne également un procédé de modélisation de la conductance électrique des parois de glandes sudoripares eccrines, comprenant la mise en oeuvre du procédé selon l'invention avec des électrodes disposées dans la région des glandes eccrines en question.

BREVE DESCRIPTION DES FIGURES

D'autres caractéristiques, buts et avantages de la présente invention apparaîtront à la lecture de la description détaillée qui va suivre, au regard des figures annexées, données à titre d'exemples non limitatifs et sur lesquelles :

- La figure 1, déjà décrite, représente schématiquement le voltammogramme d'une électrode servant d'anode et de cathode dans le procédé selon l'invention.
- La figure 2 est une représentation simplifiée de la peau humaine.
- La figure 3 représente schématiquement une glande sudorale eccrine.
- Les figures 4a et 4b illustrent la modélisation de la réponse courant-tension de la peau humaine.
- La figure 5 représente un système d'analyse utilisé dans le procédé selon l'invention.
- La figure 6 représente le biais de mesure entre le courant physiologique et le courant effectivement mesuré à l'anode.
- La figure 7 représente un exemple de courbes courant-tension mesurées à l'anode et à la cathode selon une variante du procédé.

DESCRIPTION DETAILLEE D'UN MODE DE REALISATION PREFERE

*Modélisation du* **comportement électrochimique** *de* **la peau à** *basses* **tensions**

On a conçu un modèle amélioré du comportement électrochimique de la peau soumise à un potentiel électrique. Les principaux éléments de la peau considérés dans le modèle sont décrits en référence à la figure 2.

Aux basses tensions utilisées dans le procédé selon l'invention, c'est-à-dire inférieures à 10V, la couche la plus externe de la peau P, le stratum corneum SC, est électriquement isolant. Cette couche, comprise dans l'épiderme EP, est constituée d'une matrice de lipides et de cornéocytes, c'est-à-dire de cellules mortes. Elle est traversée par des follicules pileux FP et par les follicules des glandes sudorales FGS, comme schématisé en figure 2. Alors, seuls les follicules des glandes sudorales FGS sont électriquement conducteurs.

On rappelle que deux types de glandes sudorales sont impliqués par les mesures électrochimiques. Les glandes appocrines GA sont localisées dans des zones pileuses telles que le creux axillaire, le pubis ou la poitrine, et possèdent un canal sécréteur qui débouche dans un follicule pileux. Ces glandes sont localisées dans des zones non concernées par les mesures et ne seront donc pas considérées dans la suite.

Les glandes eccrines GE, quant à elles, sont les plus nombreuses et sont présentes quasiment sur toute la surface de la peau. On en trouve en abondance (en moyenne 500 / cm$^2$) sur les paumes des mains, la plante des pieds et sur le front, là où sont réalisées les mesures. Ce modèle concerne particulièrement le comportement électrochimique des follicules des glandes eccrines.

*Loi de conservation générale*

Considérons un milieu continu : pest la densité ou la masse volumique, $\underline{U}$ est la vitesse, dans un volume matériel $\Omega(t)$ présentant une frontière $\partial\Omega(t)$ avec une normale extérieure $\underline{n}$, le volume (dépendant du temps t) que nous allons étudier et suivre dans son mouvement. On note la coordonnée spatiale (i.e. la position) $\underline{x} \in \Omega(t)$.

Soit $a(\underline{x},t)$ un champ vectoriel ou scalaire suffisamment régulier, une équation de bilan pour la quantité $(\rho,a)$ peut être écrite sous la forme générale :

$$\frac{\partial}{\partial t}(\rho \, a) + div(\rho \, a \otimes \underline{U}) = \mathcal{A} + div(A)$$

où $\mathcal{A}$ est un terme de production/disparition (volumique), $A$ est un flux d'échange (surfacique), $\otimes$ désigne le produit vectoriel usuel, et $\frac{\partial}{\partial t}$ est la dérivée temporelle partielle.

Et dans le cas présent d'un espace à une dimension (1D), on obtient:

$$\frac{\partial}{\partial t}(\rho \, a) + \frac{\partial}{\partial x}(\rho \, a \, u) = \mathcal{A} + \frac{\partial A}{\partial x}$$

Les quantités physiques classiques conservées sont : la masse, la quantité de mouvement, et l'énergie. Cette dernière est de moindre intérêt ici car elle introduit des variables additionnelles dont au moins la température. Pour les deux autres, on a, en l'absence de forces visqueuses et de gradient de pression :

| **Bilan** | $a$ | $A$ |
|---|---|---|
| Masse | 1 | 0 |
| Quantité de mouvement | $\underline{U}$ | 0 |

Où $(\mathcal{F})$ est la résultante des forces volumiques extérieures.

*Modèle de la glande*

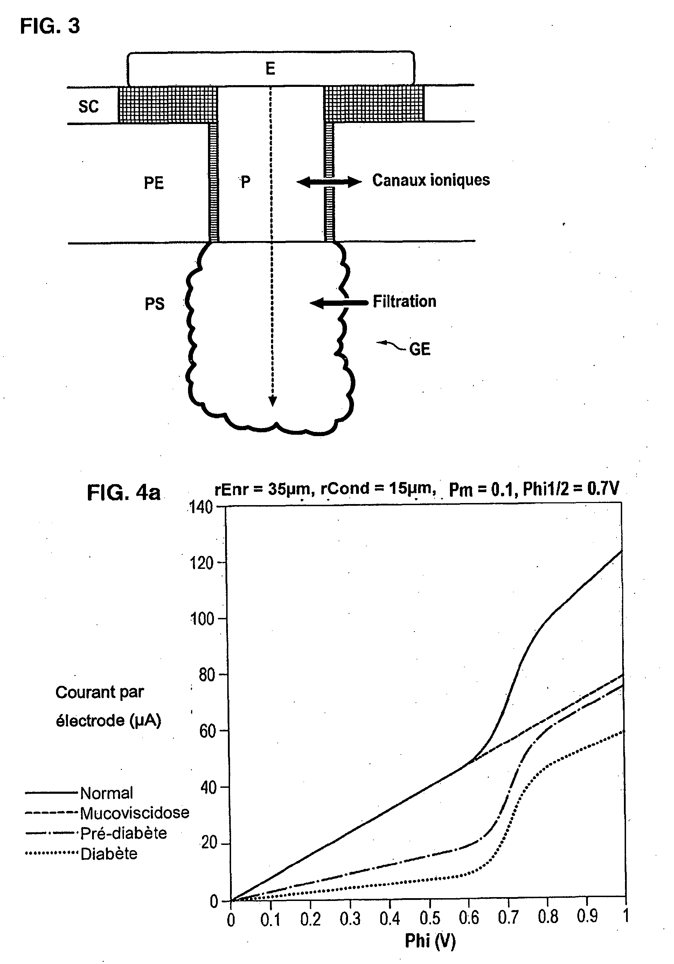

En référence à la figure 3, est illustré schématiquement un modèle d'une glande eccrine GE.

La glande eccrine GE est constituée de deux parties : la partie sécrétrice PS, où la sueur est filtrée (isotoniquement) du plasma sanguin, est un enroulement. La partie excrétrice PE, où certaines espèces peuvent se déplacer dans les deux sens au travers d'un canal ionique (absorption ou excrétion, selon leur gradient électrochimique), est un conduit presque droit qui mène à un pore P à la surface de la peau. Ces deux régions ont des longueurs du même ordre de grandeur, et l'enroulement est légèrement plus large que le conduit.

Le modèle géométrique consiste à dérouler l'enroulement et à le joindre au conduit pour former, comme dans le modèle de Chizmazdhev (voir à ce propos « Y. A. Chizmadzhev, A. V. Indenbom, P. L. Kuzmin, S. V. Galichenko, J. C.Weaver, and R. O. Potts, Electrical properties of skin at moderate voltages: Contribution of appendageal macropores, Biophys. J., vol. 74, pp. 843-856, 1998 »), un tube cylindrique.

Les principales variables sont les concentrations $c_i$ et vitesses $u_i$ des principaux ions présents dans la sueur : $i \in \{Cl^-, Na^+, H^+\}$. Nous devons ajouter le potentiel électrique à l'intérieur de la glande $\Phi$. Ils sont tous fonction de $(x, t)$, où $x$ est l'abscisse le long de l'axe normal à la surface de la peau et orienté vers l'intérieur du corps, et $t$ le temps.

Les paramètres géométriques sont :

➢ $h$: épaisseur du stratum corneum (SC),
➢ $r_e$ : rayon du conduit, et $r_s$ : rayon de l'enroulement,
➢ $L^e$, $L^s$ longueurs respectives de la partie excrétrice et de la partie sécrétrice.

Les paramètres électriques sont :

➢ $\sigma$ : conductivité de l'électrolyte (sueur),

➢ $\Phi^A$ : potentiel appliqué à l'anode,

➢ $\Phi^{ext}$ : potentiel constant atteint par le corps après application d'une tension anodique. Dans la suite et pour simplifier l'analyse et le calcul, on prend $\Phi^{ext}= 0$, i.e. ce potentiel sera choisi comme référence.

➢ Conductances surfaciques des deux parties : $G^e, G^s$. Généralement elles sont fonction de la différence de potentiel entre les deux côtés de la paroi de la glande($\Phi - \Phi^{ext}$).

Deux courants sont en présence : un courant axial le long de l'axe de la glande, dû aux mouvements des ions le long de l'axe, et des courants transversaux dus aux charges qui traversent ou s'accumulent sur la paroi de la glande.

*Courant axial*

La densité surfacique du courant axial, en fonction des principales variables, est par définition :

$$J^a = F \sum_i z_i . c_i . u_i$$

où $z_i$ est la charge de l'ion $i$ et F est la constante de Faraday (charge d'une mole). Le courant axial est ensuite donné par :

$$I^a = \pi . r^2 . J^a$$

Par ailleurs, la loi d'Ohm stipule que le courant = (la conductance * la différence de potentiel), et la conductance = (la conductivité * la surface) / la longueur, ce qui donne :

$$I^a = -\sigma . \pi r^2 \frac{\partial \Phi}{\partial x}$$

On déduit le champ électrique E qui par définition est:

$$\mathbb{E} \equiv -\frac{\partial \Phi}{\partial x} = \frac{F}{\sigma} \sum_i z_i . c_i . u_i$$

*Courant transversal au travers de la paroi*

Le courant transversal au travers de la paroi correspond aux charges qui traversent la paroi de la glande, appelé le courant de canal ionique. Il est lié aux ions $Cl^-$ et $Na^+$ qui passent au travers de la membrane épithéliale en utilisant leurs propres canaux ioniques spécifiques. L'approche du canal ionique est plus féconde qu'un simple modèle de conductance car elle prend également en compte le gradient chimique. La densité de courant $J_i^t$ pour un ion $i$ est donnée par :

$$J_i^t = z_i . G_i . P_i . (\Phi - \Phi^{ext} - \Phi_i)$$

Où $G_i$ est la conductance par unité de surface; $P_i$ le pourcentage (ou la probabilité) de canaux ouverts, dépendant des concentrations ioniques de chaque côté de la paroi, et généralement donné par une fonction de Boltzmann ; $\Phi_i$ est le potentiel à l'équilibre de l'ion selon la loi de Nernst (voir à ce propos « J. Cronin, Mathematics of Cell Electrophysiology. New York: Marcel Dekker, 1981, vol. 63, Lecture Notes in Pure and Applied Mathematics."):

$$\Phi_i = \frac{R.T}{z_i.F} . ln\left(\frac{c_i}{c_i^{ext}}\right)$$

dans laquelle $R$ est la constant des gaz parfaits et $T$ est la température absolue.

*Courant transversal capacitif*

Il correspond aux ions qui s'accumulent sur la paroi de la glande. Sa densité (par unité de surface sur la paroi de la glande) s'écrit :

$$C_w \cdot \frac{\partial \Phi}{\partial t}$$

Pour une capacité électrique $C_w$ de la paroi. Toutefois ce courant n'est pas évident à modéliser avec nos variables : $c_i, u_i$ et est transitoire (i.e. il disparaît dans les états stationnaires). Pour ces deux raisons, il ne sera pas pris en compte ici, puisqu'on s'intéresse principalement aux solutions permanentes.

*Conservation de la masse*

Nous avons vu que la conservation de la masse s'écrit :

$$\frac{\partial}{\partial t}(\rho) + \frac{\partial}{\partial x}(\rho\ u) = \mathcal{A}$$

Il reste maintenant à préciser le terme source $\mathcal{A}$ qui représente, par unité de volume, la perte ou le gain dû au courant transversal au travers de la paroi.
Mais au préalable, on rappelle que pour un ion $i$, sa densité $\rho_i$ est liée à sa concentration $c_i$ par la relation simple :

$$\rho_i = c_i.M_i$$

où $M_i$ est la masse molaire « constante » de l'ion.
On trouve:

$$\mathcal{A} = -\frac{2\ M_i}{r.F} . J_i^t$$

Finalement, avec la concentration, l'équation de conservation de la masse est donnée par :

$$\frac{\partial c_i}{\partial t} + \frac{\partial(c_i.u_i)}{\partial x} = \frac{-2}{r.F}\ . J_i^t$$

*Conservation de la quantité de mouvement*

On a vu que la conservation de la quantité de mouvement s'écrit :

$$\frac{\partial}{\partial t}(\rho\ u) + \frac{\partial}{\partial x}(\rho\ u^2) = \mathcal{F}$$

Il reste maintenant à préciser le terme source $\mathcal{F}$ qui représente, par unité de volume, la résultante des forces extérieures présentes.
On considère que les ions sont des sphères rigides se déplaçant dans un fluide continu incompressible. On rappelle que quand des espèces chargées se déplacent, elles créent un champ électrique $\mathbb{E}$ (déjà mentionné) et un champ magnétique $\mathbb{B}$. En supposant que l'agitation thermique et les interactions entre les espèces et avec la paroi sont négligeables, et que la loi de Stokes est applicable, les espèces sont soumises aux forces suivantes :

• Force de Lorentz:

$$z_i.e.\,\mathbb{E} + z_i.e.\,u_i \wedge \mathbb{B}$$

Le second terme est rigoureusement nul dans notre modèle à une dimension car il est orthogonal à la vitesse.

• La trainée due à l'opposition de la sueur:

$$-\xi_i.(u_i - v)$$

Où $v$ est la vitesse constante de la sueur et $\zeta_i$ est le coefficient de Stokes donné par:

$$\xi_i = 6\pi.\mu.H_i$$

Avec $\mu$ la viscosité dynamique de la sueur (donc de l'eau) et $H_i$ le rayon hydrodynamique de l'ion.
La force résultante est :

$$\mathcal{R}_i = z_i.e.\,\mathbb{E} - \xi_i.(u_i - v)$$

Et par unité de volume :

$$\mathcal{F}_i = \mathcal{R}_i.\frac{\rho_i}{m_i} \equiv \mathcal{R}_i.\frac{c_i.M_i}{m_i}$$

On en déduit finalement la loi de conservation de la quantité de mouvement :

$$\frac{\partial(c_i.u_i)}{\partial t} + \frac{\partial(c_i.u_i^2)}{\partial x} = \frac{z_i.e}{m_i}.c_i.\mathbb{E} - \frac{\xi_i}{m_i}.c_i.(u_i - v)$$

*Application d'une tension par une électrode*

Quand une électrode est appliquée sur la peau, elle comble le tube de sorte que la sueur physiologique et ses composants sont bloqués : la vitesse est donc nulle ($v = 0$).
L'application d'un potentiel implique que seules les espèces qui réagiront ($Cl^-$ à l'anode et $H^+$ à la cathode) commencent à accélérer vers l'électrode par électromigration. Initialement et dans un état permanent, les autres espèces sont au repos. Pour conclure, seuls $Cl^-$ et $H^+$ sont concernés.

*Simplifications : la quantité de mouvement*

A l'état stationnaire, l'équation de quantité de mouvement est réduite à :

$$c = c^* + \mathcal{C}.\left[\frac{\partial\Phi}{\partial x}.\frac{\partial}{\partial x}\left(\frac{1}{c}\right) + \frac{2}{c}.\frac{\partial^2\Phi}{\partial x^2}\right]$$

$$c^* = \frac{\xi \cdot \sigma}{e \cdot F}$$

$$C = \frac{\sigma^2 \cdot m}{F^2 \cdot e}$$

On rappelle que le coefficient de Stokes (ou coefficient de friction) est donné par:

$$\xi = 6 \cdot \pi \cdot H \cdot \mu$$

où $\mu$ est la viscosité dynamique de l'eau, et $H$ est le rayon hydrodynamique (ou de Stokes) d'un ion chlorure ou d'un proton. Ce rayon est en fait déduit de la mobilité de l'ion dans l'électrolyte, définie par :

$$\mathcal{M} = \frac{z \cdot e}{\xi}$$

Qui a été tabulée, voir à ce propos "P. W. Atkins and J. D. Paula, Elements of physical chemistry, Oxford University Press, 2005".

Afin d'obtenir certains ordres de grandeur des quantités $c^*$ and $C$, voici certaines applications numériques, d'abord avec le chlorure :

| | |
|---|---|
| $\overline{\mathcal{M}}$ (chlorure Cl⁻) | $7.91 \cdot 10^{-8}$ $m^2 \cdot s^{-1} \cdot V^{-1}$ |
| $H$ (chlorure Cl⁻) | $1\text{Å} = 10^{-10} m$ |
| $\mu$ | $0.001$ $kg/(m.s)$ |
| $\sigma$ | $0.01$ $S/cm$ |
| $e$ | $1.602176 \cdot 10^{-19} C$ |
| $F$ | $96485$ $C/mol$ |
| $m = \dfrac{M}{N}$ | $\dfrac{35 \cdot 10^{-3} \, kg/mol}{6.0221415 \cdot 10^{23} \, /mol}$ |

En sachant que pour l'unité Coulomb :

$$C = A.s = V.S.s = \frac{J}{V} = \frac{1}{V} \cdot \frac{kg \cdot m^2}{s^2} = \frac{10^4}{V} \cdot \frac{kg \cdot cm^2}{s^2}$$

On obtient :

| | |
|---|---|
| $c^*$ | $1.21935 \cdot 10^{-4} \dfrac{\text{mol}}{\text{cm}^3} \approx 120 \dfrac{\text{mmol}}{\text{L}} = c^{ext}$ |
| $C$ | $3.896 \cdot 10^{-25}$ |

Si l'on prend $c = c^* = Cte$, on commet une $erreur \approx \frac{2C}{c^*} \cdot \frac{\partial^2 \Phi}{\partial x^2}$. Comme $\frac{\partial^2 \Phi}{\partial x^2} = \frac{2C}{r \cdot \sigma} \cdot \Phi$ avec les ordres de grandeur suivants :

| G | $10^{-6}$ $S/cm2$ |
|---|---|
| r | 0.001 $cm$ |
| Φ | $1V$ |

On trouve :

$erreur \approx 16.10^{-22}$ $mol/cm3$.

Ainsi, il apparaît clairement que le deuxième terme à droite $C_r$ est absolument négligeable, ce qui mène à une distribution constante de la concentration le long de l'axe, égale à celle à l'interstisium. Ce simple résultat est remarquable et simplifie l'équation de la masse car le potentiel à l'équilibre $\Phi_{\acute{e}}$ y sera rigoureusement nul.

Et pour le proton, M = 36.23 puis $H$ = 2.3459.$10^{-11}m$, ce qui donne:

$$c^* = 28.15 \frac{mmol}{L},$$

$pH$ = 1.55

Ainsi, avec le proton, il existe une discontinuité de contact à l'extrémité de l'enroulement.

Finalement, on déduit la vitesse, à partir du potentiel $\Phi$ (voir section prochaine), par la loi d'Ohm :

$$u = M . \frac{\partial \Phi}{\partial x}$$

Avec la mobilité déjà définie (ratio de la vitesse de dérive de la particule sur le champ électrique appliqué) :

$$M = \frac{\sigma}{c^* . F} = \frac{e}{\xi}$$

*Simplification : la masse*

Pour le proton, il n'existe pas de canal dédié, il s'agit d'un passe-partout. Ainsi $P_i$ = 1 $et$ $\Phi_i$ = 0.

Pour les chlorures, on vient de voir que dans un état permanent, $c_i(x)$ = $Cte$ = $c^{ext}$ ce qui implique que le potentiel à l'équilibre (potentiel de Nernst) est rigoureusement nul : $\Phi_i$ = 0.

Dans tous les cas, à l'état stationnaire, la conservation de la masse est réduite à une équation différentielle ordinaire pour le potentiel :

$$\frac{d^2\Phi}{dx^2} = \frac{2}{r.\sigma} . P . G . \Phi$$

Ainsi, on observe un découplage total entre les équations : d'abord, la conservation de la masse permet d'obtenir le potentiel, ensuite la conservation de la quantité de mouvement donne la concentration et la vitesse (section précédente).

Dans la suite, on suppose que les conductances sont constantes, et que la dépendance par rapport au potentiel est assurée grâce à la probabilité d'ouverture d'un canal et/ou la fonction d'électroporation $P(\Phi)$.

*Cas de probabilités constantes*

Dans le cas de probabilités constantes P=1, la précédente équation, différentielle ordinaire d'ordre 2, devient à coefficients constants, et a la solution générale classique :

$$\Phi(x) = C_1 \cdot e^{\gamma x} + C_2 \cdot e^{-\gamma x}$$

où $\gamma = \sqrt{\dfrac{2 \cdot G}{r \cdot \sigma}}$ et $c_1, c_2$ sont des constantes à déterminer, en fonction des conditions aux frontières qui sont :

- Surface : application d'une tension $\Phi^A$ et continuité du courant axial entre SC et le conduit :

$$\frac{\partial \Phi}{\partial x}(0) = \frac{\Phi(0) - \Phi^A}{h}$$

- Raccord entre le conduit excréteur et l'enroulement sécréteur : continuité du potentiel et du courant *en x = -L^s*.
- Intérieur (extrémité de la glande, frontière avec l'interstice) : condition de Neumann (continuité du courant qui est nul) en *x = X* :

$$\frac{\partial \Phi(X)}{\partial x} = 0$$

Cette solution que nous pouvons construire de manière analytique est essentielle. C'est celle qui donne la conductance électrochimique de la peau telle que mesurée par le système d'analyse électrophysiologique utilisé dans la présente invention. En effet, la mesure est réalisée avant tout décollement de pente physiologique et donc pour une probabilité toujours constante.

Plus précisément, on prouve que la conductance mesurée vaut :

$$\mathbb{C} \approx 2\pi \cdot r_e \cdot L_e \cdot G_e + 2\pi \cdot r_s \cdot L_s \cdot G_s$$

Cette simple relation est remarquable : la conductance électrochimique de la peau mesurée est la somme de la conductance "réelle totale" du conduit et de conductance "réelle totale" de l'enroulement.

En outre, elle ne dépend ni de l'épaisseur du stratum corneum h, ni de la conductivité de la sueur $\sigma$.

*Prise en compte de l'ouverture du canal ionique et de l'électroporation*

La probabilité d'ouverture d'un canal de chlorure est donnée par une loi de Boltzmann :

$$P = p_{min} + \frac{p_{max} - p_{min}}{1 + e^{-\frac{z \cdot F}{R \cdot T}(\Phi - \Phi^{ext} - \Phi_{1/2})}}$$

Avec :

| | |
|---|---|
| $p_{min}$ | Probabilité minimale : 0 - 0.1 |
| $p_{max}$ | Probabilité maximale $\approx 1$ |
| $z$ | Charge apparente pour $Cl^- \approx 1$ |
| $\Phi_{1/2}$ | Potentiel de demi-activation(P = 0.5) à ajuster |

On a représenté en figure 4a à titre d'exemple la fonction de Boltzmann pour trois valeurs de z différentes.

On combine la loi de Boltzmann pour l'ouverture de canal avec un modèle d'électroporation similaire à celui de Chizmazdhev. Dans ce dernier, la conductance du conduit varie exponentiellement en fonction de la tension, c'est un modèle déduit de la réponse du stratum corneum à haute tension. L'électroporation est prise en compte ici grâce à une multiplication de la probabilité précédente par :

$$\exp\left[\alpha * \left\{\max(\Phi - \Phi^{ext} - \Phi_{1/2}, 0)\right\}^2\right]$$

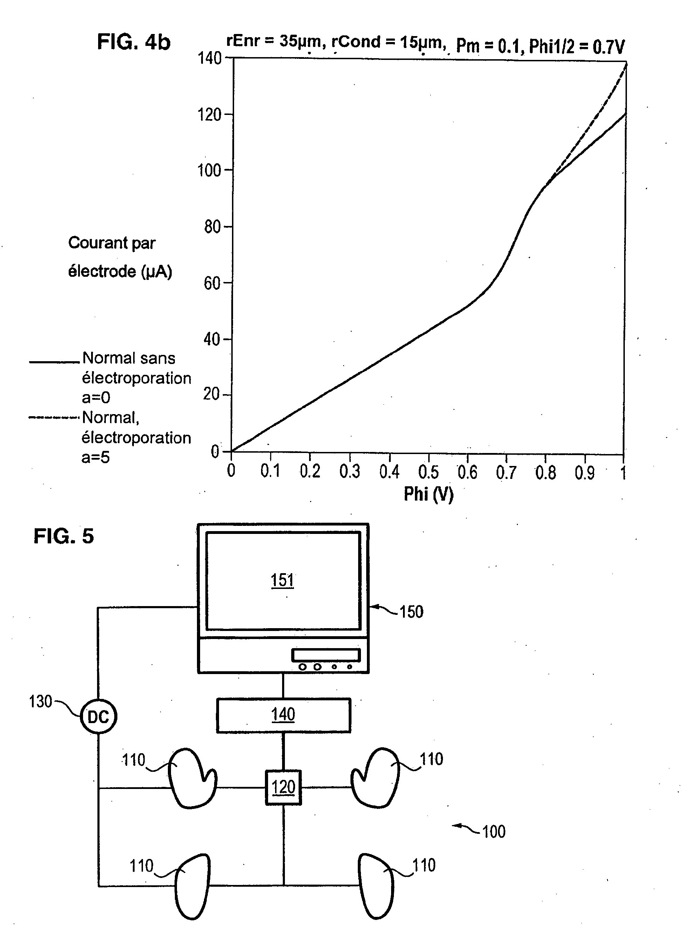

qui dépend d'un paramètre $\alpha$ à choisir, de l'ordre de 1. On a représenté à cet effet en figure 4b la fonction d'électroporation pour deux $\alpha$ différents.

*Solution numérique*

La prise en compte de l'ouverture des canaux et de l'électroporation amène à définir la fonction non-linéaire $P(\Phi)$.

Ceci mène à résoudre l'équation non linéaire différentielle ordinaire (EDO) :

$$\frac{d^2 \cdot \Phi}{dx^2} = \frac{2}{r \cdot \sigma} \cdot G \cdot P(\Phi) \cdot \Phi$$

sujette aux mêmes conditions aux frontières que précédemment (voir la section « Probabilités constantes »).

Donc, cette EDO doit satisfaire les mêmes conditions aux frontières à plus d'une valeur : une à la surface, l'autre à l'extrémité de la glande. Le problème qui en résulte n'est plus l'intégration classique d'une EDO ou problème aux valeurs initiales de Cauchy, mais est appelé un problème aux limites en deux points

La solution adoptée est une méthode de tir. L'intégration de l'EDO par l'algorithme de deuxième ordre de Stoermer procède depuis la surface jusqu'à l'extrémité de la glande, et on essaie de faire correspondre la condition aux limites de courant nul à la fin de l'intégration. Le point clé ici est cette correspondance délicate. Elle est mise en oeuvre en utilisant la méthode globalement convergente de Newton-Raphson.

*Application numérique*

En référence aux figures 4a et 4b sont représentées des courbes courant-tension pour une glande obtenues selon le modèle.

Les principaux paramètres qui régulent la réponse électrique de la glande eccrine sont les conductances (surfaciques) de la partie sécrétrice et de la partie excrétrice. On considère des valeurs typiques correspondant aux cas suivants : patient normal, patient atteint de mucoviscidose, patient atteint de diabète. Les ordres de grandeurs des conductances sont donnés en $\mu S/cm^2$.

| Conductance | Normal | Mucoviscidose | Diabète |
|---|---|---|---|
| Enroulement $G^s$ | 1-2 | 1-2 | 0.1-0.2 |
| Conduit $G^e$ | 1-2, P : 0.1- 1 | 1-2 P=$P_{min}$=0.1 | 1-2 P:0.1 - 1 |

Concernant la géométrie de la glande, les parties excrétrice et sécrétrice ont quasiment le même diamètre et la même longueur, ici en cm :

| **Taille** | Longueur | Rayon |
|---|---|---|
| Enroulement | 0.1 - 0.5 | 0.003 - 0.004 |
| Conduit | 0.1 - 0.5 | 0.001 |

On considère encore les paramètres suivants :

| Densité de glandes | 500 / cm2 |
|---|---|
| Surface effective de contact avec l'électrode | 30 cm2 |
| Longueur du conduit et de l'enroulement | 0.2 cm |
| Rayon du conduit | 0.0015 cm |

(suite)

| Rayon de l'enroulement | 0.0035 cm |
|---|---|
| Probabilité min | 0.1 |
| Conductivité de la sueur | 0.01 S / cm |
| Epaisseur du SC | 0.004 cm |

On obtient, en figure 4a, la modélisation des courbes courants-tension pour différents types de patients (sain, diabétique, « pré-diabétique », atteint de mucoviscidose), et prenant en compte les probabilités d'ouverture des canaux. En figure 4b, cette modélisation est réalisée pour un patient sain, en prenant ou non en compte l'électroporation.

On constate finalement que la caractéristique courant-tension en régime permanent de la peau humaine présente initialement une partie linéaire avant un décollement de la pente (ou une déviation de linéarité) qui provient de la dépendance non linéaire de la conductance surfacique de la glande / tube par rapport à la tension due à l'ouverture des canaux et/ou à l'électroporation.

*Procédé d'analyse électrophysiologique*

Le modèle exposé ci-avant indique que la mesure de la conductance de la peau permet de connaître rigoureusement la conductance des parois des glandes eccrines, c'est-à-dire la capacité de la glande à sécréter des ions. Ainsi, quand on applique un potentiel sur ces glandes on évalue exactement la fonction sudorale qui est impactée dans certaines maladies telles que le diabète ou la mucoviscidose.

On met à profit ce résultat dans le procédé selon l'invention, dans lequel on mesure la conductance électrochimique de la peau. Pour ce faire, on se munit d'un système d'analyse électrophysiologique 100 schématisé en figure 5.

Ce système 100 comprend une pluralité d'électrodes 110, dont au moins deux électrodes pour les pieds (pied gauche, pied droit), deux électrodes pour les mains (main gauche, main droite), et deux électrodes pour le front (partie gauche du front, partie droite du front).

Alternativement, il ne comprend que quatre électrodes 110, pour les mains et les pieds.

Typiquement, avec un système à quatre électrodes tel que décrit ci-dessus, on effectue les mesure avec les paires d'électrodes suivantes (désignation abrégée entre parenthèses) :

| Anode | Cathode |
|---|---|
| Main gauche (MG) | Main droite (MD) |
| Main droite (MD) | Main gauche (MG) |
| Pied gauche (PG) | Pied droit (PD) |
| Pied droit (PD) | Pied gauche (PG) |

Ces électrodes sont de préférence de grandes dimensions, i.e. leur surface est comprise entre 50 et 200 cm$^2$, afin qu'elles recouvrent toute la surface de la zone analysée. Ces électrodes, une fois appliquées sur la peau, sont soumises à potentiel permettant l'établissement des phénomènes électrochimiques étudiés dans le modèle ci-avant.

Pour être mesurable par une électrode, le courant dû au transport d'ions de la sueur dans la glande doit être inférieur au courant que peut transmettre l'électrode et qui est dû au transfert d'électrons entre l'électrode et la sueur.

Cette condition est assurée ici par l'utilisation d'électrodes sensibles, constituées de matériaux tels que le nickel ou l'acier inoxydable, qui permettent de visualiser les phénomènes même à basses tensions (< 10 V).

Pour appliquer un potentiel à la peau, ces électrodes sont connectées à une source de tension continue ajustable 130, adaptée pour délivrer des créneaux de tension continue.

Le système comprend également un circuit de commutation 120. Ce circuit peut sélectivement connecter une ou plusieurs électrodes en haute impédance, et connecter une paire d'autres à la source de tension. Ces dernières sont des électrodes dites actives, car elles appliquent le potentiel sur la peau afin de réaliser les mesures.

Le système comprend en outre un circuit de mesure 140, qui relève des données représentatives du courant et du potentiel dans les électrodes actives, et des potentiels sur au moins une électrode connectée en haute impédance. Ce circuit de mesure peut également comprendre ou être connecté à un processeur 150, adapté pour traiter les données, et le cas échéant les afficher sous forme de courbe sur un afficheur 151.

La mesure se fait successivement et indépendamment sur les mains et les pieds, en alternant gauche et droite. A chaque fois, deux électrodes sont actives : l'anode de potentiel $V^a$ positif imposé et la cathode de potentiel $V^c$ mesuré, le courant entre les deux est aussi mesuré.

En outre, au moins une autre électrode passive est reliée à la masse en haute impédance. De préférence, c'est le cas des deux électrodes passives, notées $XG$ et $XD$ (×=main ou pied, D=droite, G=gauche), de potentiels $v^{XG}$ et $V^{XD}$ mesurés, permettant de récupérer le potentiel atteint par le corps $V^x \approx V^{XG} \approx V^{XD}$.

Ainsi on mesure traditionnellement la conductance électrochimique de la peau à l'anode et à la cathode, en divisant le courant entre les électrodes par le potentiel respectivement à l'anode et à la cathode, retranché du potentiel d'une électrode haute impédance et éventuellement d'une correction d'électrode.

Dans le cas d'une pluralité d'électrodes connectées en haute impédance, on calcule le potentiel moyen pour déterminer la conductance électrochimique de la peau à l'anode et à la cathode.

Au cours dé l'étape de mesure, la source de tension délivre à l'anode un ou plusieurs créneaux de tension, d'une durée supérieure ou égale à 0.2 seconde. De préférence, la durée cumulée de l'ensemble des créneaux est comprise entre 5 secondes et une minute, et préférablement comprise entre 10 et 30 secondes. Cette durée est suffisamment longue pour permettre l'établissement de phénomènes électrochimiques dans la peau.

Les créneaux ont un potentiel compris entre 1 et 4 V. La source de tension peut appliquer à l'anode plusieurs créneaux, de durée égale et de tension variable d'un créneau à l'autre, par exemple croissante ou décroissante entre 1 et 4 V.

Alternativement, elle peut simplement appliquer un unique créneau de tension comprise entre 1 et 4 V, de préférence entre 3 et 3.5 V.

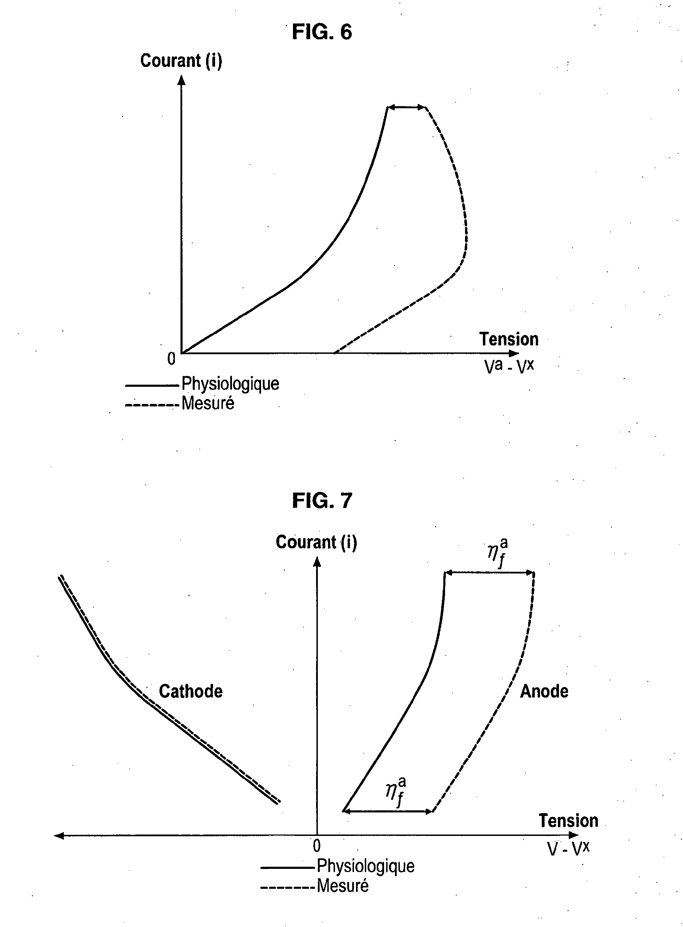

Comme décrit en préambule, l'oxydation de l'anode génère au niveau de celle-ci une surtension qui évolue de façon croissante en fonction du temps et du potentiel appliqué à l'anode. A la cathode en revanche, la réduction entraine une décroissance de la surtension jusqu'à annulation de celle-ci. Ces surtensions ont pour effet de fausser la mesure, comme visible sur la figure 6.

Dans cette figure, la courbe en trait plein représente le courant physiologique à mesurer, qui doit normalement passer par l'origine. La courbe en trait pointillé représente le courant effectivement mesuré, en fonction de la différence entre le potentiel à l'anode et le potentiel du corps, ce dernier étant mesuré par les électrodes reliées en haute impédance.

Le procédé selon l'invention permet de corriger cette mesure.

A cet effet, on utilise le principe de régénération des électrodes, consistant à connecter une électrode en tant que cathode afin de supprimer toute surtension.

Pour ce faire, le procédé selon l'invention présente au moins une étape additionnelle, préalable à l'étape de mesure, au cours de laquelle on régénère au moins une électrode, en l'utilisant en tant que cathode, avant de la connecter en haute impédance.

Au cours d'une telle étape de régénération, la cathode est soumise à un potentiel continu compris entre -1 et -4 V, et de préférence entre -3 et -3.5 V. Ce potentiel résulte de l'application à l'anode d'une tension continue, sous forme d'un créneau unique, ou de plusieurs créneaux de tensions variables ou non d'un créneau à l'autre.

De préférence, la durée totale de l'application de tension est supérieure au temps de régénération de la cathode, compris entre quelques secondes et quelques dizaines de secondes. Par exemple, elle peut être comprise entre 5 s et 1 minute, ou préférablement entre 10 et 30 secondes.

Le fait qu'une électrode haute impédance soit régénérée permet de garantir que le potentiel mesuré à cette électrode corresponde exactement au potentiel atteint par le corps à cette zone. Cela garantit que la différence de potentiel entre les électrodes actives et l'électrode régénérée permet de déterminer exactement le potentiel, et donc la conductance électrochimique de la peau au niveau des électrodes actives.

Le procédé peut en outre comprendre une étape de régénération supplémentaire d'une ou plusieurs autres électrodes qui sont connectées en haute impédance au cours de l'étape de mesure. Dans ce cas, on calcule la moyenne de leurs potentiels pour obtenir le potentiel atteint par le corps.

Bien entendu, il est possible d'utiliser une étape de régénération également en tant qu'étape de mesure. Dans ce cas le circuit de mesure relève les potentiels aux électrodes connectées en haute impédance, le potentiel à la cathode, et le courant entre l'anode et la cathode.

Enfin, le procédé peut exploiter les méthodes explicitées ci-après pour supprimer les biais dans l'étape de mesure.

*Méthode du cycle performant*

Cette méthode consiste à réaliser un cycle d'étapes successives de régénération et de mesures exploitant le fait qu'une électrode utilisée en tant que cathode dans l'un ou l'autre type d'étape (mesure ou régénération) est régénérée. Ce cycle comprend donc :

- au moins une étape de régénération d'une électrode haute impédance, au cours de laquelle ladite électrode, connectée en haute impédance dans l'étape de mesure, est branchée en tant que cathode,
- une étape de régénération d'une anode, au cours de laquelle l'anode de l'étape de mesure est branchée en tant que cathode,

- et une étape de mesure.

De nombreux cycles peuvent être imaginés. On peut envisager un cycle au cours duquel la cathode utilisée au cours d'une étape de mesure est utilisée en tant qu'anode au cours d'une étape de mesure subséquente.

Alternativement, on crée un cycle au cours duquel on mesure la conductance électrochimique de la peau sur les mains et sur les pieds à partir du potentiel de l'anode (conductance liée aux ions chlorure) ; il faut donc réaliser au moins quatre mesures où l'anode se situe aux quatre sites différents : main droite, main gauche, pied droit, pied gauche.

Un cycle possible, dans lequel les deux électrodes connectées en haute impédance et l'anode sont toujours régénérées, est le suivant :

| Anode - Cathode | Nature de l'étape | Remarques |
|---|---|---|
| MG - PG | Régénération | PG régénérée après |
| MG - PD | Régénération | PD régénérée après |
| MG - MD | Régénération | MD régénérée après |
| MD - MG | Mesure | Anode régénérée au début, MG régénérée après |
| MG - MD | Mesure | Anode régénérée au début, MD régénérée après |
| PG - MG | Régénération | MG régénérée après |
| PD - PG | Mesure | Anode régénérée au début, PG régénérée après |
| PG - PD | Mesure | Anode régénérée au début |

Au cours de ce cycle, les anodes sont toujours régénérées, et le « zéro » est assuré pour les deux électrodes connectées en haute impédance ; il en résulte que les circuits de mesure gauche et droite sont symétriques.

Bien entendu, la portée du procédé n'est pas limité à ce cycle, ni à l'utilisation de quatre électrodes seulement. Son principe est transposable aisément à d'autres cycles ou à un nombre plus important d'électrodes, par exemple six.

_Méthode de la mesure de la conductance par la pente de la courbe_

De retour à la figure 6, on constate que la conductance linéaire du courant physiologique n'est autre que la pente de la partie droite du courant mesuré.

On peut donc calculer la conductance linéaire du courant physiologique quand par détermination de la pente de la courbe quand de faibles tensions sont appliquées à l'anode, une fois que la surtension côté anode et cathode est stabilisée.

Une autre méthode d'exploitation de la pente de la courbe consiste à estimer la surtension à l'anode en extrapolant le potentiel quand le courant s'annule, puis à déterminer la conductance (linéaire ou pas) de la peau au niveau de l'anode en divisant le courant par la différence entre le potentiel de l'anode retranchée de cette surtension, et le potentiel d'une électrode régénérée connectée en haute impédance.

_Mesure de la conductance par une correction d'électrode_

Outre la détermination précédente de la surtension à partir de la pente, la conductance peut être estimée aussi par une correction d'électrode.

En variante, le procédé selon l'invention peut comprendre une étape intermédiaire, au cours de laquelle une surtension est estimée par une différence de potentiel entre une électrode régénérée connectée en haute impédance, et une autre électrode, non régénérée, connectée en haute impédance. Pendant cette mesure, la source de tension continue délivre toujours à l'anode un ou plusieurs créneaux de tension de même tension et même durée que décrit précédemment. La différence de potentiel ainsi mesurée permet de déterminer la surtension quand cette électrode non régénérée servira d'anode au cours d'une étape de mesure ultérieure.

On obtient une estimation de la conductance électrochimique de la peau à l'anode en divisant le courant entre l'anode et la cathode par la différence entre le potentiel de l'anode corrigé (i.e. auquel on a retranché la surtension) et le potentiel de l'électrode régénérée connectée en haute impédance.

*Méthode de duplication de la mesure*

En variante, le procédé selon l'invention comprend une étape de régénération supplémentaire, comprise entre l'étape de régénération et l'étape de mesure, au cours de laquelle l'anode et la cathode sont respectivement les mêmes que dans l'étape de mesure.

Au cours de cette étape, la cathode est complètement régénérée, tandis que la surtension à l'anode croit pour atteindre l'offset final.

Par conséquent, comme visible sur la figure 7, au cours de l'étape de mesure subséquente, la courbe mesurée coté cathode, en traits pointillés, est confondue avec la courant physiologique, en trait plein. En revanche, à l'anode, la surtension est constante et égale à l'offset final $\eta_f^a$, i.e. le courant mesuré en traits pointillés et le courant physiologique en traits pleins sont simplement décalés de l'offset. Ceci permet une évaluation précise de toute la courbe à l'anode et à la cathode : à la fois la partie de conductance linéaire, et la partie de décollement.

En effet, dans les plages de tensions pour lesquelles la conductance de la peau est linéaire, on peut estimer cette conductance à l'anode, comme à la cathode, en mesurant la pente de la courbe courant-tension.

Par ailleurs, on peut également déterminer la conductance de la peau au niveau de la partie de décollement, à l'anode comme à la cathode.

A la cathode, il suffit de diviser le courant entre les électrodes par le potentiel à la cathode, retranché du potentiel d'une électrode haute impédance régénérée.

A l'anode, on divise le courant entre les électrodes par à la différence de potentiel entre l'anode et une électrode haute impédance régénérée, à laquelle on a retranché la surtension à l'anode.

Grâce à l'invention, l'homme du métier saura mettre en oeuvre des procédés de mesure précis de la conductance électrochimique de la peau et pourra en déduire la présence chez un patient de dysfonctionnements ou de pathologies, tels que par exemple la mucoviscidose ou une neuropathie autonomique.

**Revendications**

**1.** Procédé d'analyse électrophysiologique mise en oeuvre dans un système comprenant :

une série d'électrodes, destinées à être placées en différentes régions du corps humain,
une source de tension continue, commandée pour engendrer des créneaux de tension continue,
un circuit de commutation, agencé pour sélectivement relier une paire d'électrodes dites actives à la source de tension, lesdites électrodes actives constituant une anode et une cathode, et pour connecter au moins une autre électrode passive en haute impédance servant à mesurer le potentiel atteint par le corps, et
un circuit de mesure agencé pour relever des données représentatives du courant dans les électrodes actives et des potentiels sur au moins certaines électrodes connectées en haute impédance en réponse à l'application des créneaux, lesdites données permettant de déterminer une valeur de la conductance électrochimique de la peau,
le procédé comprenant au moins une étape de mesure au cours de laquelle la source de tension continue ajustable applique à l'anode une série desdits créneaux de tension continue, et au cours de laquelle le circuit de mesure relève lesdites données,
le procédé étant **caractérisé en ce qu'**il comprend en outre une étape préalable à l'étape de mesure, au cours de laquelle une électrode qui est connectée en haute impédance au cours de l'étape de mesure est régénérée en étant connectée à la source de tension en tant que cathode.

**2.** Procédé d'analyse électrophysiologique selon la revendication 1, dans lequel, au cours de l'étape de régénération, la cathode est soumise à un potentiel continu compris entre -1 et -4 V, préférablement entre -3 et -3.5 V.

**3.** Procédé d'analyse électrophysiologique selon l'une des revendications 1 ou 2, dans lequel, au cours de l'étape de régénération, la source de tension délivre un créneau de tension d'une durée comprise entre 5 secondes et une minute, préférablement entre 10 et 30 secondes ou des créneaux de tension identique ou variable d'un créneau à l'autre, d'une durée cumulée comprise entre 5 secondes et une minute, préférablement entre 10 et 30 secondes.

**4.** Procédé d'analyse électrophysiologique selon la revendication 1, comprenant une étape de régénération supplémentaire, préalablement à l'étape de mesure, au cours de laquelle une autre électrode connectée en haute impédance ou l'électrode utilisée en tant qu'anode au cours de l'étape de mesure est régénérée en étant connectée à la source de tension en tant que cathode.

**5.** Procédé d'analyse électrophysiologique selon l'une des revendications 1 à 4, comprenant en outre une étape intermédiaire, entre l'étape de régénération et l'étape de mesure, au cours de laquelle on mesure une différence de tension entre une électrode non régénérée connectée en haute impédance, et une électrode régénérée connectée en haute impédance, ladite différence permettant de déterminer une valeur de surtension à l'électrode non régénérée, et dans lequel, au cours de l'étape de mesure, on connecte en tant qu'anode l'électrode non régénérée dont on a mesuré la surtension.

**6.** Procédé d'analyse électrophysiologique selon la revendication 5, comprenant une étape de détermination, à partir de la surtension de l'électrode haute impédance non régénérée, d'une correction à appliquer aux valeurs mesurées au cours de l'étape de mesure.

**7.** Procédé d'analyse électrophysiologique selon la revendication 6, dans lequel l'électrode régénérée connectée en haute impédance au cours de l'étape intermédiaire a été préalablement connectée en tant que cathode, et comprenant une étape dans laquelle on retranche du potentiel mesuré à l'anode au cours de l'étape de mesure la surtension déterminée au cours de l'étape intermédiaire.

**8.** Procédé d'analyse électrophysiologique selon l'une des revendications 1 à 4, comprenant une étape de régénération supplémentaire, entre l'étape de régénération et l'étape de mesure, au cours de laquelle l'anode et la cathode sont respectivement les mêmes que celles de l'étape de mesure.

**9.** Procédé d'analyse électrophysiologique selon les revendications 1 à 4 ou 8, dans lequel la conductance électrochimique de la peau déterminée à l'anode ou à la cathode est la pente de la courbe mesurée dans un graphe courant-tension pour des tensions appliquées à l'anode inférieure à 2 V, indépendamment de toute surtension.

**10.** Procédé d'analyse électrophysiologique selon la revendication 8, dans lequel la conductance de la peau au niveau de la cathode est obtenue en divisant le courant mesuré à la cathode par la différence de potentiel entre la cathode et une électrode régénérée connectée en haute impédance.

**11.** Procédé d'analyse électrophysiologique selon la revendication 8, dans lequel on estime une surtension à l'anode par la valeur du potentiel à l'anode extrapolé quand le courant s'annule, et on obtient la conductance de la peau au niveau de l'anode en divisant le courant mesuré à l'anode par la différence entre le potentiel de l'anode retranché de ladite surtension et le potentiel d'une électrode régénérée connectée en haute impédance.

**12.** Procédé d'analyse électrophysiologique selon l'une des revendications 1 à 4, dans lequel la durée cumulée des créneaux de tension appliqués lors de l'étape de mesure est comprise entre 5 secondes et une minute, préférablement entre 10 et 30 secondes.

**13.** Procédé d'analyse électrophysiologique selon la revendication 12, dans lequel la durée cumulée des créneaux de tension appliqués lors de l'étape de mesure est supérieure ou égale à la durée du créneau appliqué lors d'une étape de régénération.

**14.** Procédé d'analyse électrophysiologique selon l'une des revendications 1 à 13, dans lequel on mesure une conductance de la peau à l'anode en divisant le courant mesuré à l'anode par la différence de potentiel entre l'anode retranché de la surtension et une électrode régénérée connectée en haute impédance, et dans lequel la conductance électrochimique de la peau ainsi obtenue correspond à la conductance électrique des parois des glandes sudoripares eccrines en contact avec les électrodes actives.

**15.** Procédé de modélisation de la conductance électrique des parois de glandes sudoripares eccrines, comprenant la mise en oeuvre du procédé selon l'une des revendications 1 à 13 avec des électrodes disposées dans la région des glandes eccrines en question.

**Patentansprüche**

**1.** Elektrophysiologisches Analyseverfahren, das in einem System umgesetzt wird, das umfasst:

eine Reihe von Elektroden, die zur Platzierung in verschiedenen Regionen des menschlichen Körpers bestimmt sind,

eine Gleichspannungsquelle, die gesteuert wird, um Gleichspannungrechtecke zu erzeugen,

einen Kommunikationschaltkreis, der ausgebildet ist, um selektiv ein aktives Elektrodenpaar mit der Spannungsquelle zu verbinden, wobei die aktiven Elektroden eine Anode und eine Kathode darstellen, und um mindestens eine andere passive Elektrode, die zur Messung des vom Körper erreichten Potentials dient, in Hochimpedanz zu verbinden, und

einen Messkreis, der ausgebildet ist, um repräsentative Daten des Stroms in den aktiven Elektroden und der Potentiale auf mindestens bestimmten Elektroden, die in Hochimpedanz verbunden sind, als Antwort auf die Anwendung des rechtecke zu ermitteln, wobei die Daten erlauben, einen Wert der elektrochemischen Leitfähigkeit der Haut zu bestimmen,

wobei das Verfahren mindestens einen Messschritt umfasst, bei dem die einstellbare Gleichspannungsquelle auf die Anode eine Reihe des Gleichspannungsrechtecke anwendet und bei dem der Messkreis die Daten ermittelt,

wobei das Verfahren **dadurch gekennzeichnet ist, dass** es ferner einen Schritt vor dem Messschritt umfasst, bei dem eine Elektrode, die während des Messschritts in Hochimpedanz verbunden ist, als Kathode mit der Spannungsquelle verbunden regeneriert wird.

2. Elektrophysiologisches Analyseverfahren nach Anspruch 1, wobei die Kathode während des Regenerationsschritts einem kontinuierlichen Potential zwischen -1 und -4 V, vorzugsweise zwischen -3 und -3,5 V, inklusive ausgesetzt wird.

3. Elektrophysiologisches Analyseverfahren nach einem der Ansprüche 1 oder 2, wobei die Spannungsquelle während des Regenerationsschritts ein Spannungsrechteck einer Dauer zwischen 5 Sekunden und einer Minute, vorzugsweise zwischen 10 und 30 Sekunden, inklusive oder Rechtecke identischer oder von einem Rechteck zum anderen variabler Spannung einer kumulierten Dauer zwischen 5 Sekunden und einer Minute, vorzugsweise zwischen 10 und 30 Sekunden, inklusive bereitstellt.

4. Elektrophysiologisches Analyseverfahren nach Anspruch 1, das vor dem Messschritt einen zusätzlichen Regenerationsschritts umfasst, bei dem eine andere, in Hochimpedanz verbundene Elektrode oder die während des Messschritts als Anode verwendete Elektrode als mit der Spannungsquelle als Kathode verbunden regeneriert wird.

5. Elektrophysiologisches Analyseverfahren nach einem der Ansprüche 1 bis 4, das ferner einen Übergangsschritt zwischen dem Regenerationsschritt und dem Messschritt umfasst, bei dem eine Spannungsdifferenz zwischen einer nicht regenerierten, in Hochimpedanz verbundenen Elektrode und einer regenerierten, in Hochimpedanz verbundenen Elektrode gemessen wird, wobei die Differenz die Bestimmung eines Überspannungswerts an der nicht regenerierten Elektrode erlaubt, und wobei während des Messschritts die nicht regenerierte Elektrode, deren Überspannung gemessen wurde, als Anode verbunden wird.

6. Elektrophysiologisches Analyseverfahren nach Anspruch 5, das ausgehend von der Überspannung der nicht regenerierten Hochimpedanz-Elektrode einen Bestimmungsschritt einer an die während des Messschritts gemessenen Werte anzuwendenden Korrektur umfasst.

7. Elektrophysiologisches Analyseverfahren nach Anspruch 6, wobei die regenerierte, während des Übergangsschritts in Hochimpedanz verbundene Elektrode zuvor als Kathode verbunden war und einen Schritt umfasst, bei dem vom an der Anode während des Messschritts gemessenen Potential die während des Übergangsschritts gemessene Überspannung abgezogen wird.

8. Elektrophysiologisches Analyseverfahren nach einem der Ansprüche 1 bis 4, das zwischen dem Regenerationsschritt und dem Messschritt einen zusätzlichen Regenerationsschritt umfasst, bei dem die Anode und die Kathode jeweils dieselben wie beim Messschritt sind.

9. Elektrophysiologisches Analyseverfahren nach den Ansprüchen 1 bis 4 oder 8, wobei, unabhängig von jeder Überspannung, die an der Anode oder an der Kathode bestimmte elektrochemische Leitfähigkeit der Haut die Steigung der in einem Strom-Spannungs-Graph für an die Anode unter 2 V angelegte Spannungen gemessene Kurve ist.

10. Elektrophysiologisches Analyseverfahren nach Anspruch 8, wobei die Leitfähigkeit der Haut im Bereich der Kathode durch Division des an der Kathode gemessenen Stroms durch die Potentialdifferenz zwischen der Kathode und einer regenerierten, in Hochimpedanz verbundenen Elektrode ermittelt wird.

**11.** Elektrophysiologisches Analyseverfahren nach Anspruch 8, wobei eine Überspannung an der Anode durch den Wert des extrapolierten Potentials an der Anode geschätzt wird, wenn der Strom zusammenfällt, und man die Leitfähigkeit der Haut im Bereich der Anode durch Division des an der Anode gemessenen Stroms durch die Differenz zwischen dem Potential der Anode minus Überspannung und dem Potential einer regenerierten, in Hochimpedanz verbundenen Elektrode erhält.

**12.** Elektrophysiologisches Analyseverfahren nach einem der Ansprüche 1 bis 4, wobei die kumulierte Dauer der während des Messschritts angewendeten Spannungs Rechteck zwischen 5 Sekunden und einer Minute, vorzugsweise zwischen 10 und 30 Sekunden, inklusive ist.

**13.** Elektrophysiologisches Analyseverfahren nach Anspruch 12, wobei die kumulierte Dauer der während des Messschritts angewendeten Spannungs Rechtecke größer oder gleich der Dauer des während eines Regenerationsschritts angewendeten Rechteckes ist.

**14.** Elektrophysiologisches Analyseverfahren nach einem der Ansprüche 1 bis 13, wobei eine Leitfähigkeit der Haut an der Anode durch Division des an der Anode gemessenen Stroms durch die Potentialdifferenz zwischen der Anode minus Überspannung und einer regenerierten, in Hochimpedanz verbundenen Elektrode gemessen wird, und wobei die derart erhaltene elektrochemische Leitfähigkeit der Haut der elektrischen Leitfähigkeit der Wände der ekkrinen Schweißdrüsen im Kontakt mit den aktiven Elektroden entspricht.

**15.** Verfahren für die Modellierung der elektrischen Leitfähigkeit der Wände ekkrinen Schweißdrüsen, das die Umsetzung des Verfahrens nach einem der Ansprüche 1 bis 13 mit Elektroden, die in der Region der fraglichen ekkrinen Drüsen angeordnet sind, umfasst.

**Claims**

**1.** An electrophysiological analysis method applied in a system comprising:

a series of electrodes, intended to be placed in different regions of the human body,
a DC voltage source, controlled for increasing the DC voltage square wave pulses,
a switching circuit, laid out for selectively connecting a pair of so-called active electrodes to the voltage source, said active electrodes forming an anode and a cathode, and for connecting at least one of the passive electrode in high impedance being used for measuring the potential attained by the body, and
a measuring circuit laid out for measuring representative data of the current in the active electrodes and potentials on at least certain electrodes connected in high impedance in response to the application of the square waves, said data allowing determination of a value of the electrochemical conductance of the skin,
the method comprising at least one measuring step during which the adjustable voltage source applies to the anode a series of said road DC voltage square waves, and during which the measuring circuit measures said data, the method being **characterized in that** it further comprises a step prior to the measuring step, during which an electrode which is connected in high impedance during the measuring step is regenerated by being connected to the voltage source as a cathode.

**2.** The electrophysiological analysis method according to claim 1, wherein, during the regeneration step, the cathode is subject to a DC potential comprised between -1 and -4 V, preferably between -3 and -3.5 V.

**3.** The electrophysiological analysis method according to one of claims 1 or 2, wherein, during the regeneration step, the voltage source delivers a voltage square wave pulse with a duration comprised between 5 seconds and one minute, preferably between 10 and 30 seconds or square wave pulses with an identical or variable voltage from one pulse to the other, the cumulative duration of which is comprised between 5 seconds and one minute, preferably between 10 and 30 seconds.

**4.** The electrophysiological analysis method according to claim 1, comprising an additional regeneration step prior to the measuring step, during which another electrode connected in high impedance or the electrode used as an anode during the measuring step is regenerated while being connected to the voltage source as a cathode.

**5.** The electrophysiological analysis method according to one of claims 1 to 4, further comprising an intermediate step, between the regeneration step and the measuring step, during which a voltage difference is measured between a non-regenerated electrode connected in high impedance and a regenerated electrode connected in high impedance, said difference allowing determination of an overvoltage value at the non-regenerated electrode, and wherein, during the measuring step, the non-regenerated electrode, the overvoltage of which has been measured, is connected as an anode.

6.  The electrophysiological analysis method according to claim 5, comprising a step for determining from the overvoltage of the non-regenerated high impedance electrode, a correction to be applied to the values measured during the measuring step.

7.  The electrophysiological analysis method according to claim 6, wherein the regenerated electrode connected in high impedance during the intermediate step was connected beforehand as a cathode, and comprising a step in which the overvoltage determined during the intermediate step is subtracted from the potential measured at the anode during the measuring step.

8.  The electrophysiological analysis method according to one of claims 1 to 4, comprising an additional regeneration step, between the regeneration step and the measuring step, during which the anode and the cathode are respectively the same as those of the measuring step.

9.  The electrophysiological analysis method according to claims 1 to 4 or 8, wherein the electrochemical conductance of skin determined at the anode or at the cathode is the slope of the curve measured on a current-voltage graph for applied voltages at the anode of less than 2 V, independently of any overvoltage.

10. The electrophysiological analysis method according to claim 8, wherein the conductance of the skin at the cathode is obtained by dividing the current measured at the cathode by the potential difference between the cathode and a regenerated electrode connected in high impedance.

11. The electrophysiological analysis method according to claim 8, wherein and overvoltage at the anode is estimated by the value of the potential at the anode extrapolated to when the current becomes zero, and the conductance of the skin at the anode is obtained by dividing the current measured at the anode by the difference between the potential of the anode subtracted with said overvoltage and the potential of a regenerated electrode connected in high impedance.

12. The electrophysiological analysis method according to one of claims 1 to 4, wherein the cumulative duration of the square wave voltage pulses applied during the measuring step is comprised between 5 seconds and one minute, preferably between 10 and 30 seconds.

13. The electrophysiological analysis method according to claim 12, wherein the cumulative duration of the square wave voltage pulses applied during the measuring step is greater than or equal to the duration of the applied square wave during a regeneration step.

14. The electrophysiological analysis method according to one of claims 1 to 13, wherein a conductance of the skin at the anode is measured by dividing the current measured at the anode by the potential difference between the anode, the overvoltage having been subtracted therefrom, and a regenerated electrode connected in high impedance, and wherein the thereby obtained electrochemical conductance of the skin corresponds to the electric conductance of the walls of eccrine sweat glands in contact with the active electrodes.

15. A method for modeling the electric conductance of the walls of eccrine sweat glands, comprising the application of the method according to one of claims 1 to 13 with electrodes positioned in the region of the relevant eccrine glands.

## FIG. 1

## FIG. 2

## FIG. 3

## FIG. 4a

rEnr = 35µm, rCond = 15µm, Pm = 0.1, Phi1/2 = 0.7V

Courant par électrode (µA)

——— Normal
------ Mucoviscidose
—·—· Pré-diabète
············· Diabète

Phi (V)

**FIG. 4b**

rEnr = 35µm, rCond = 15µm, Pm = 0.1, Phi1/2 = 0.7V

Courant par électrode (µA)

———— Normal sans électroporation a=0

------- Normal, électroporation a=5

Phi (V)

**FIG. 5**

## FIG. 6

Courant (i)

Tension
$V^a - V^x$

0

——— Physiologique

- - - - - Mesuré

## FIG. 7

Courant (i)

$\eta_f^a$

Cathode

Anode

$\eta_f^a$

Tension
$V - V^x$

0

——— Physiologique

- - - - - Mesuré

24

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2912893

**Littérature non-brevet citée dans la description**

- **GIN H et al.** Non-invasive and quantitative assessment of sudomotor function for peripheral diabetic neuropathy evaluation. *Diabetes Metab,* 2011
- **Y. A. CHIZMADZHEV ; A. V. INDENBOM ; P. L. KUZMIN ; S. V. GALICHENKO ; J. C.WEAVER ; R. O. POTTS.** Electrical properties of skin at moderate voltages: Contribution of appendageal macropores. *Biophys. J.,* 1998, vol. 74, 843-856
- Lecture Notes in Pure and Applied Mathematics. **J. CRONIN.** Mathematics of Cell Electrophysiology. Marcel Dekker, 1981, vol. 63
- **P. W. ATKINS ; J. D. PAULA.** Elements of physical chemistry. Oxford University Press, 2005